Europäisches Patentamt

European Patent Office

Office européen des brevets

(19)

(11) **EP 0 826 367 A2**

(12) ## DEMANDE DE BREVET EUROPEEN

(43) Date de publication:
04.03.1998 Bulletin 1998/10

(51) Int Cl.⁶: **A61K 7/48**, A61K 35/78

(21) Numéro de dépôt: **97401946.5**

(22) Date de dépôt: **19.08.1997**

(84) Etats contractants désignés:
**AT BE CH DE DK ES FI FR GB GR IE IT LI LU MC NL PT SE**

(30) Priorité: **22.08.1996 FR 9610356**

(71) Demandeur: **LVMH RECHERCHE
92752 Nanterre (FR)**

(72) Inventeurs:
• **Bonte, Frédéric
92400 Courbevoie (FR)**
• **Dumas, Marc
92700 Colombes (FR)**

(74) Mandataire: **Giraud, Françoise et al
Cabinet Beau de Loménie
158, rue de l'Université
75340 Paris Cedex 07 (FR)**

(54) **Utilisation d'un extrait de Bertholletia dans le domaine cosmétique ou pharmaceutique, et pour la préparation de milieux de cultures de cellules**

(57) L'invention concerne de nouvelles utilisations d'un extrait de Bertholletia dans le domaine cosmétique ou pharmaceutique, notamment dermatologique et pour la préparation de milieux de culture de cellules.

Elle concerne plus particulièrement les utilisations dans le domaine cosmétique où pharmaceutique ou l'on cherche à stimuler la synthèse du collagène VII et/ou à favoriser l'incorporation de la vitamine C dans les cellules de la peau, en particulier dans les fibroblastes.

Elle concerne également de nouveaux milieux de culture de cellules contenant un extrait de Bertholletia.

EP 0 826 367 A2

## Description

La présente invention concerne de nouvelles utilisations, en particulier dans le domaine cosmétique ou-pharmaceutique, notamment dermatologiquc, d'un extrait de Bertholletia.

Elle concerne également de nouvelles utilisations du même extrait dans des milieux de culture de cellules, notamment des milieux de culture de cellules de la peau humaine.

La plante Bertholletia appartient à la famille des Lecythidacées. Il s'agit d'un genre qui comprend deux espèces extrêmement voisines : Bertholletia excelsa et Bertholletia nobilis. Ce sont des arbres des régions tropicales de l'Amérique du Sud, que l'on rencontre en particulier au Brésil. Ils peuvent atteindre 30 à 40 mètres de hauteur. On les appelle communément noyers ou châtaigniers du Brésil. Les noix des deux espèces sont à peu près identiques et sont pratiquement confondues. Elles sont utilisées pour leurs qualités alimentaires.

On en trouve une description notamment dans l'ouvrage "Dictionnaire des Huiles Végétales" par Paul H. Mensier, Editions Paul Lechevalier, Paris, 1957.

En Amazonie, l'écorce du tronc de Bertholletia excelsa, qui contient notamment des saponines, est utilisée en infusion contre les maladies du foie. L'huile de ses graines peut servir à la fabrication de savons (R.E. Schultes et al. The healing forest. Discorides Press, Portland, Oregon, USA, 1990, vol. 2, pages 225-226).

Il a maintenant été découvert que les extraits de Bertholletia présentaient un grand intérêt dans le domaine cosmétique ou pharmaceutique, notamment en dermatologie, du fait de leur action sur la synthèse du collagène VII et également du fait de leur effet sur le transport de la vitamine C vers les cellules.

On sait que la peau contient essentiellement du collagène I, protéine synthétisée par les fibroblastes, cellules majoritaires du derme. Cette protéine joue un rôle de soutien et est responsable des qualités biomécaniques du derme, en particulier de sa fermeté et du maintien de son architecture (E.U. KUCHARZ, "The collagens : Biochemistry, and pathophysiology", Springer Verlag, Berlin 1992). Par ailleurs, il a été montré que les fibroblastes du derme des personnes âgées sécrètent moins de collagène que ceux des sujets jeunes (M. DUMAS et al, Mech, Ageing Dev. (1994) 73, 179-187). Ainsi, avec l'âge, se produit une diminution des qualités rhéologiques de la peau et de sa réponse aux contraintes auxquelles elle est soumise quotidiennement. La peau se distend, réagit moins bien aux tensions, perd son tonus et les rides se creusent.

On sait aussi que le collagène IV est un collagène majeur de la jonction dermo-épidermique. On pourra se rapporter à ce sujet à l'article de Briggaman R.A., intitulé "Biochemical composition of the epidemial-demial junction and other basement membranes", publié dans J. Invest. Dermatol. 78 (1982), pages 1-6, ou l'article de P. Verando intitulé "La jonction dermoépidermique" publiée dans le Séminaire Inserm, vol. 214, (1991), pages 83-100, édition Inserm,

Ce collagène IV qui se trouve au niveau de la lamina densa, intervient dans l'attachement cellulaire et est synthétisé par les kératinocytes. Les plaques d'ancrage assurent les liaisons entre les fibrilles d'ancrage liées aux kératinocytes et la matrice extracellulaire du derme constituée de fibres de collagène (collagène I et III). Ces plaques d'ancrage sont constituées de collagène IV. Elles sont essentielles dans le maintien d'une interface fonctionnelle entre les deux compartiments cutanés. Le collagène IV joue également un rôle important dans les processus de cicatrisation. Il permet la formation d'une peau cicatricielle de bonne qualité.

Le collagène de type VII est le constituant prédominant des fibrilles d'ancrage, associées à la membrane basale, reliant l'épiderme au derme. Il est synthétisé par les kératinocytes basaux et, en quantité moindre, par les fibroblastes du derme comme décrit par R.BURGESON, "Type VII collagen, anchoring fibrils, and epidermolysis bullosa", J.Invest. Dermatol, 101,252-255,1993. Des études récentes ont montré qu'une application topique d'acide rétinoique augmentait le nombre de fibrilles d'ancrage sur des peaux ayant subi un vieillissement actinique.

Selon Y.Q. CHEN, A.MAUVIEL, J.RYYNANEN, S.SOLLBERG, J.UITTO : "Type VII collagen gene expression by human skin fibroblasts and keratinocytes in culture : Influence of donor age and cytokine responses", J.Invest.Dermatol (1994), 102, (2), 205-209, les manifestations du vieillissement cutané, telles qu'une fragilité cutanée accrue et une diminution des capacités de réparation de l'épiderme, pourraient être attribuables à une synthèse de collagène VII diminuée chez les sujets âgés.

On a maintenant mis en évidence de façon tout à fait surprenante l'effet d'un extrait de Berthollctia sur la sécrétion de collagène VII par une souche de kératinocytes humains normaux en culture.

Cette constatation a conduit à la mise au point de nouvelles compositions cosmétiques ou pharmaceutiques, notamment dermatologiques, plus particulièrement utiles dans toutes les applications où l'un cherche à stimuler la synthèse du collagène VII, en vue en particulier de favoriser la cohésion derme-épiderme. Cette propriété s'est avérée particulièrement utile pour l'élaboration de compositions topiques cosmétiques ou dermatologiques. De telles compositions permettent, en particulier, de favoriser la cohésion derme-épiderme dans les peaux atones, relâchées. Elles permettent également de traiter les pathologies où la jonction dermo-épidermique est déficiente, telle que l'épidermolyse bulleuse.

Par ailleurs, on a également montré que, de façon surprenante, les extraits de Bertholletia permettaient d'améliorer considérablement l'incorporation (ou transport) de vitamine C dans les cellules cutanées, en particulier les fibroblastes.

Or, il était connu que la vitamine C ou acide L-ascorbique intervenait à différents niveaux dans les cellules de mammifères :

- comme agent anti-oxydant grâce à son potentiel redox faible, en particulier dans les mécanismes de protection cellulaire contre les peroxydes produits continuellement par la chaîne respiratoire mitochondriale,
- comme co-facteur d'enzymes telles que les hydroxylases essentielles à la synthèse des collagènes de type I et III,

Ainsi, la vitamine C présente une grande importance dans plusieurs fonctions vitales de la cellule, importance qui nous explique l'intérêt porté à son transport depuis le milieu extracellulaire, jusqu'au compartiment cytoplasmique. Or, cette molécule n'est pas synthétisée par les cellules humaines, notamment cutanées. Elle est apportée par l'alimentation, absorbée au niveau intestinal et véhiculée vers tous les tissus par le sang dans lequel le taux plasmatique atteint en général 25 μM environ. Dans la cellule, son incorporation s'effectue contre le gradient de concentration protéique par un transporteur protéique à haute affinité.

Cette dernière constatation a permis la mise au point de nouvelles compositions cosmétiques ou pharmaceutiques, notamment dermatologiques, plus particulièrement utiles dans toutes les applications où l'on cherche à améliorer l'incorporation de la vitamine C dans les cellules cutanées, en particulier dans les fibroblastes.

Ainsi, selon l'une de ses caractéristiques essentielles, l'invention concerne l'utilisation d'un extrait de Bertholletia, en particulier de Bertholletia excclsa, comme agent cosmétique destiné à stimuler la synthèse du collagène VII et/ou à favoriser l'incorporation de la vitamine C dans les cellules de la peau, en particulier dans les fibroblastes.

Cet agent cosmétique sera introduit dans une composition cosmétique comprenant un véhicule cosmétiquement acceptable dans lequel l'extrait sera dissous ou mis en suspension.

Suivant le premier aspect selon lequel l'agent cosmétique agit pour favoriser la synthèse du collagène VII, la composition s'avérera particulièrement utile dans toutes les applications où l'on cherche à améliorer la cohésion derme-épiderme. Il pourra en particulier s'agir d'un produit anti-ridcs, d'un produit destiné à lutter contre le vieillissement actinique de la peau, c'est-à-dire le vieillissement induit par les rayonnements, en particulier le rayonnement solaire ultra-violet.

D'une façon générale, selon ce premier aspect, les compositions cosmétiques de l'invention s'avèrent particulièrement utiles en tant que produits raffermissants de la peau destinés, en particulier, à lutter contre les peaux lâches ou atones.

Selon le deuxième aspect, les compositions cosmétiques pourront être également destinées à améliorer l'incorporation de la vitamine C par les cellules de la peau. Un tel effet conduira à une amélioration du métabolisme cellulaire, en particulier à une stimulation des synthèses cellulaires, notamment celles des différents types de collagène et de l'élastine. Ainsi, en favorisant l'incorporation de la vitamine C, les extraits de Bertholletia, en particulier de Bertholletia excelsa, améliorent l'état de la peau, notamment en restaurant ou en augmentant sa tonicité, son élasticité et en retardant l'apparition des rides.

Selon une variante de l'un ou l'autre de ces deux aspects, ledit agent cosmétique est incorporé dans une composition à usage topique contenant en outre de la vitamine C ou un dérivé d'acide ascorbique.

L'invention vise également l'utilisation des mêmes extraits de Bertholletia pour la préparation d'une composition pharmaceutique destinée à traiter les pathologies liées à une déficience de la jonction derme-épiderme, en particulier celles liées à une déficience de la synthèse du collagène VII.

A titre d'exemple de telles pathologies, on citera, en particulier, l'épidermolyse bulleuse.

Selon une autre variante de l'invention, l'extrait de la plante Bertholletia sera utilisé pour la préparation d'une composition pharmaceutique, notamment dermatologique, destinée à traiter les pathologies liées à une déficience de l'incorporation de la vitamine C dans les cellules de la peau, en particulier dans les fibroblastes.

La composition pharmaceutique ci-dessus contiendra avantageusement un véhicule pharmaceutiquement acceptable dans lequel l'extrait de Bertholletia sera dissous ou mis en suspension.

Elle contiendra également, selon une variante particulièrement avantageuse, en outre de la vitamine C ou un dérivé d'acide d'ascorbique. L'effet de l'extrait de Bertholletia dans une telle composition sera d'améliorer l'incorporation de la vitamine C dans les cellules déficientes en un tel composé.

Selon une variante, dans les différentes compositions cosmétiques ou pharmaceutiques visées par la présente invention, l'extrait de Bertholletia est un extrait d'écorce de cette plante, en particulier un extrait de tronc de la plante Bertholletia excelsa.

Selon une autre variante de réalisation, l'extrait de Bertholletia est un extrait des fruits, c'est-à-dire un extrait des noix, de Bertholletia. On choisira de préférence un extrait de péricarpe des noix de Bertholletia excelsa.

En particulier, l'extrait de Bertholletia peut être obtenu selon le procédé décrit ci-après à titre indicatif, mais nullement limitatif.

On effectue une première extraction de l'écorce ou des noix, en particulier l'écorce du tronc ou du péricarpe de noix, de la plante par un solvant polaire, avantageusement choisi parmi le groupe constitué par l'eau, les alcools comportant de préférence de 1 à 4 atomes de carbone, les solvants chlorés comportant de préférence 1 à 2 atomes de carbone, les esters organiques comportant de préférence de 3 à 6 atomes de carbone et les solvants mixtes à base d'un mélange quelconque des solvants précités.

Encore de préférence, le solvant de première extraction est choisi parmi le groupe consistant de l'eau, du méthanol, de l'éthanol, d'un mélange méthanol-eau ou un mélange éthanol-eau, du chloroforme et du dichlorométhane. Encore de préférence, il s'agit de l'eau, du méthanol, de l'éthanol ou d'un mélange quelconque de ces solvants.

Un extrait tout particulièrement préféré selon l'invention sera un extrait aqueux du péricarpe de noix de Bertholletia, et plus particulièrement du Bertholletia excelsa.

Le rapport de l'écorce ou des noix, en particulier le péricarpe de noix, à l'agent d'extraction n'est pas critique et généralement sera compris entre 1 : 5 et 1 : 20 parties en poids.

L'extraction s'effectue généralement à des températures comprises entre la température ambiante et le point d'ébullition du solvant utilisé pour l'extraction.

De préférence, cette première extraction est effectuée au reflux sous pression atmosphérique pendant une durée de 2 à 4 h. En outre, elle est avantageusement précédée d'une macération à froid pendant 2 à 4 h dans le solvant d'extraction.

En fin d'extraction, la phase du solvant contenant l'extrait est filtrée puis concentrée et/ou évaporée à sec sous pression réduite. On obtient ainsi un premier extrait brut de Bertholletia selon l'invention. Cet extrait brut peut être purifié selon divers procédés bien connus de l'homme de l'art.

Les extraits de Bertholletia, plus particulièrement de Bertholletia excelsa, sont riches en saponines. De plus, ces extraits, en particulier les extraits de péricarpe de noix de Bertholletia excelsa, quelque soit le solvant polaire d'extraction utilisé, tel que l'eau ou le méthanol, contiennent des tanins, en particulier des tanins éllagique et galliques.

Si l'on souhaite enrichir en saponines l'extrait de Bertholletia selon l'invention, on opère selon le procédé décrit ci-après à titre indicatif, mais nullement limitatif. L'extrait obtenu après la première extraction est évaporé à sec. Le résidu est ensuite repris par du méthanol. La solution obtenue est versée dans de l'acétone. Il se forme alors un précipité que l'on récupère par filtration. De préférence, ce précipité est séché, puis dialysé contre de l'eau déminéralisée. Enfin, on lyophilise pour obtenir un extrait sec de Berthollctia enrichi en saponines, selon l'invention.

Les procédés d'extraction décrits ci-dessus s'appliquent en particulier à l'espèce Bertholletia excelsa.

Dans l'un ou l'autre des aspects précédents, on utilisera de préférence l'extrait de Berthollctia, en particulier Bertholletia excelsa, à une concentration comprise entre 0,0001 % et 1 % en poids par rapport au poids total de la composition finale. De préférence, cette concentration est comprise entre 0,01 % et 0,25 % en poids par rapport au poids total de la composition finale.

Egalement dans l'un quelconque des aspects précédents, la composition selon l'invention contient de préférence en outre une substance active choisie parmi le groupe constitué par l'acide mallécassique, l'acide asiatique, le madécassoside, l'asiaticosidc, l'alpha- 1-protéinase inhibiteur, les inhibiteurs de collagénase, tels que l'acide rétinoique, les inhibiteurs d'élastase, la lysine, la proline, le 2-oxoglutarate, le ginsénoside $R_0$, la vitamine D et ses dérivés, les ecdystéroïdes, en particulier la bêta-ecdysone et la vitamine E.

Enfin, selon un dernier aspect, l'invention concerne également un procédé de traitement de cellules en culture, en particulier de cellules de la peau humaine, notamment de fibroblastes et de kératinocytes, par une concentration efficace d'un extrait de Bertholletia pour obtenir une stimulation de la synthèse du collagène VII ou une amélioration de l'incorporation de la vitamine C dans lesdites cellules.

Le procédé de traitement de cellules en culture précité, selon l'invention, est particulièrement avantageux lorsqu'il est appliqué dans le cas de la préparation d'une peau reconstituée. Immobilisées sur un support, lesdites cellules reconstituent plus rapidement une matrice extracellulaire, et l'apparition d'une jonction dermo-épidermique est favorisée.

Suivant une variante préférée de mise en oeuvre de ce procédé, on traite la culture de cellules avec un extrait de Berthollctia à une concentration comprise entre 0,1 µg/ml et 10 µg/ml par rapport au milieu de culture.

D'autres buts, caractéristiques et avantages de l'invention apparaîtront clairement à la lecture de la description explicative qui va suivre, faite en référence à plusieurs exemples donnés uniquement à titre d'illustration et qui ne sauraient par conséquent en aucune façon limiter la portée de l'invention.

Dans les exemples, les pourcentages sont exprimés en poids, sauf indication contraire. Dans le cas des extraits, les quantités de ceux-ci sont exprimées en poids sec.

Exemple 1

Préparation d'un extrait méthanolique d'écorce du tronc de Bertholletia excelsa, enrichi en saponines

On prépare de la poudre d'écorce de tronc de Bertholletia excelsa par broyage. On fait ensuite macérer 49 g de cette poudre pendant 2 h dans 500 ml de méthanol. On porte le tout à reflux pendant 3 h, puis on laisse refroidir et on filtre sur verre fritté. Ce filtrat obtenu constitue un premier extrait selon l'invention, dit extrait $I_1$. Ce premier extrait est évaporé à sec. Le résidu, pesant 14,7 g, est repris par 100 ml de méthanol. On effectue une précipitation en versant cette solution dans 500 ml d'acétone, puis on filtre. Le précipité est ensuite séché sur un agent déshydratant solide tel que de la potasse, on obtient ainsi 1,33 g de produit sec. Une quantité de 830 mg de ce produit est ensuite dialysée pendant 4 jours contre 9 ml d'eau déminéralisée, puis on lyophilise. On obtient ainsi 182 mg d'extrait enrichi en saponines selon l'invention, dit extrait $I_2$.

Exemple 2

Préparation d'un extrait méthanolique de péricarpe de noix de Bertholletia excelsa du Brésil.

On rassemble les péricarpes de noix de Bertholletia excelsa du brésil disponibles dans le commerce que l'on broie grossièrement et finement.

On prend alors 100 g de la poudre de péricarpe de noix ainsi obtenue et on l'extrait successivement trois fois par 1 l de méthanol au reflux pendant 30 min.

Chaque extrait est filtré sur un filtre de diamètre de porc de 0,45 μm et les trois extraits sont réunis.

On concentre à l'aide d'un évaporateur rotatif jusqu'à obtention d'un film sec.

Ce film est repris sous agitation par de l'eau. Le lait ainsi obtenu est lyophilisé.

On obtient ainsi environ 5 g d'extrait de péricarpe de noix riche en saponines selon l'invention, dit extrait $I_3$. On notera que le rendement de l'extraction est voisin de 5 %.

Exemple 3

Préparation d'un extrait aqueux

On extrait trois fois 30 minutes par de l'eau à ébullition du péricarpe de noix préalablement broyé, on rassemble les 3 extraits, on les filtre sur 0,45 μm et on les lyophilise. On obtient de fines et légères paillettes brunes.

Le rapport initial drogue broyée / solvant est de 1/10.

Le rendement d'extraction est de 8 %.

Exemple 4

Mise en évidence de la stimulation de la synthèse du collagène VII

Le test ci-dessous a été réalisé sur l'extrait $I_3$ récupéré dans l'exemple 2.

Les tests ont été réalisés en aveugle.

1 - Protocole du test

a) Provenance des kératinocytes :

Les cultures de kératinocytes humains normaux (KHN) sont établies à partir d'un prélèvement chirurgical de peau saine. Dans la présente étude, les tests ont été réalisés sur une souche cellulaire provenant d'un lifting d'une femme caucasienne de 44 ans (souche notée KH 44 ans). Les résultats ont été confirmés sur une autre souche cellulaire notée KH 56 ans, provenant d'un lifting réalisé sur une femme caucasienne de 56 ans.

b) Conditions de culture :

Les kératinocytes sont maintenus dans le milieu SFM (Scrum Free Medium) complet (noté SFMc, GIBCO). Les cellules ont été sous-cultivées une fois à partir de la primoculture (soit un passage, noté P1).

c) Conditions de traitement :

L'ensemencement des cellules est réalisé en plaque de culture à 96 puits à raison de 30 000 KHN par puits dans le milieu SFMc. Après 24 h d'incubation nécessaire à la bonne adhérence des cellules, le milieu est remplacé par un milieu SFMc dilué à 2 %, limitant la prolifération des kératinocytes. Les solutions-mère de produit obtenu selon l'exemple 2 (noté $I_3$ dans le tableau 1) sont préparées extemporanément dans le DMSO aux concentrations de 1-2,5-5mg/ml et introduites dans le milieu d'étude à 0,1% V/V final (soit les concentrations testées ; 1 - 2,5 - 5 μg/ml). Le témoin reçoit l'excipient du produit, soit 0,1 % V/V de DMSO. Le test de viabilité XTT, ainsi que l'observation microscopique des cellules, n'ont pas révélé d'effets cytotoxiques du produit aux concentrations inférieures à 10 µg/ml. (Kit XTT BOEHRINGER, Réf. 1465015).

Les cellules sont mises au contact du milieu de traitement pendant 72 h, temps requis pour une synthèse optimale de collagène VII d'après une étude cinétique préalable.

Les surnageants d'incubation sont prélevés en vue du dosage du collagène VII sécrété. Un dosage des protéines est effectué sur le tapis cellulaire restant dans les puits (méthode BCA, SIGMA), dans le but de rapporter les quantités de collagène VII sécrétées aux taux de protéines cellulaires.

Six cultures sont réalisées pour chacune des trois concentrations et pour l'essai témoin.

d) <u>Dosage ELISA du collagène VII :</u>

Le protocole de dosage du collagène VII par une méthode ELISA a été adapté à partir de celui utilisé pour le dosage du collagène I (M. DUMAS, C. CHAUDAGNE, F. BONTE, A. MEYBECK : "In vitro biosynthesis of type I and III collagens by human dermal fibroblasts from donors of increasing age". Mechanisms of Ageing and Development, 73 (1994) 179-187).

Les modifications suivantes ont été apportées :

- 1er anticorps : Anticorps monoclonal de souris anti-collagène type VII humain, isotype IgG1 (Life Technologies, Réf. 12073-011).
- 2ème anticorps : Anticorps de chèvre anti-IgG totaux de souris, couplé à la phosphatase alcaline (Interchim, Réf. 115-056-062).

e) <u>Expression des résultats et interprétation statistique :</u>

En l'absence de collagène de type VII humain commercialement disponible pour l'établissement d'une gamme-étalon, les résultats de la sécrétion de collagène VII par les kératinocytes sont exprimés en unités de densité optique, auxquelles on soustrait l'extrait témoin du dosage (notées D.O. - Blanc). Ces valeurs sont ramenées au taux de protéines cellulaires du puits correspondant (pour 72 h d'incubation).

L'activité du produit est évaluée par le pourcentage de stimulation ;

[(Collagène VII des KHN traités - Collagène VII des KHN témoins) / Collagène VII des KHN témoins] x 100.

Les résultats obtenus sur les cultures traitées et témoins sont comparés par le test de Student non apparié, le seuil de significativité retenu étant p<0,05.

<u>Résultats - Conclusion</u>

Les résultats sont donnés dans le tableau 1 ci-dessous, à partir de la moyenne des mesures sur les différentes cultures :

Tableau 1

| Produit et concentrations | Collagène VII (DO) /100µg protéines | % d'activité | Significativité |
|---|---|---|---|
| témoin DMSO | 0,324+/-0,03 | | |
| $I_3$ 1µl/mg | 0,522 +/- 0,056 | + 61 | s |
| $I_3$ 2,5 µg/ml | 0,603 +/- 0,155 | + 86 | s |
| $I_3$ 5 µg/ml | 0,656 +/- 0,116 | + 102 | s |

s = significatif (test de Student, p < 0,05)

Il ressort clairement de ces résultats que l'extrait de Bertholletia excelsa $I_3$ stimule très fortement la synthèse du collagène VII, constituant principal des fibrilles d'ancrage. Ainsi, les extraits de Bertholletia peuvent donc être avantageusement utilisés pour améliorer la jonction dermo-épidermique, dans des compositions cosmétiques, dermatologiques ou des milieux de culture cellulaire.

Exemple 5

Mise en évidence de l'effet sur le transport de l'acide ascorbique

5.1. Matériels et méthodes

*a. Provenance des fibroblastes*

Les cultures de fibroblastes humains normaux (FHN) sont obtenues par la méthodes des explants, à partir de prélèvements de peau chirurgicalc saine. L'étude a porté sur une souche de fibroblastes provenant d'un lifting réalisé sur une femme caucasienne de 53 ans.

*b. Conditions de culture*

Les fibroblastes sont cultivés dans du milieu E 199 (Gibco), complémenté en L-Glutamine 2mM (noté E199c) et avec 10 % v/v de sérum de veau foetal (Gibco) noté SVF. La souche de fibroblastes utilisée était au stade de la primoculture.

*c. Préparation des solutions mère de produits*

L'extrait méthanolique du péricarpe de noix de Bertholletia excelsa codé $I_3$ obtenu dans l'exemple 2 a été testé en aveugle en comparaison avec son excipient de solubilisation. Des solutions mères de l'extrait aux concentrations de 5 - 2,5 et 1 mg/ml dans le diméthylsulfoxyde (DMSO) ont été préparées extemporanément. Chaque solution mère a été introduite à 0,1 % v/v dans le milieu d'incubation des cellules pour obtenir des concentrations finales de 5 - 2,5 et 1 µg/ml au contact des cellules. Ces concentrations ne présentent aucune cytotoxicité sur FHN telle que déterminée par le test au XTT basé sur la mesure de l'activité de la succinate deshydrogénase mitochondriale.

*d. Conditions de traitement des fibroblastes*

130 000 FHN sont ensemencés par puits de microplaques (24 puits FALCON) dans un milieu E 199c + 10 % SVF. Après 24 h d'incubation, le milieu est remplacé par un milieu E199C sans SVF, additionné du produit à tester, aux concentrations indiquées, ou de l'excipient de ces produits (DMSO à 0,1 % v/v) . Des témoins sans DMSO ont également été réalisés afin de vérifier l'absence d'effet propre de ce produit sur la fonction cellulaire étudiée.
Au bout de 48 heures d'incubation, on réalise l'essai sur le transport intracellulaire de la vitamine C, au moyen du scl de sodium de la vitamine C marquée (L-ascorbate-$^{14}$C de sodium).

*e. Conditions d'étude du transport de l'acide L-ascorbique-$^{14}$C dans les fibroblastes*

Les puits sont rincés avec du milieu E199C à 37°C sans sérum et l'on ajoute 0,5 ml de ce même milieu contenant 150 µmol/1 d'ascorbate-$^{14}$C de sodium (Amersham réf. : CF620, activité spécifique de 14,1 Ci/µmol) préparé extemporanément.
Les plaques sont ensuite incubées 2 heures à 37°C, puis le milieu d'incubation est retiré et le tapis cellulaire rincé avec du milieu sans sérum pour enlever toute trace de radioactivité cxtracellulaire. On ajoute ensuite 500µl/puits de méthanol à 60 % afin de perméabiliser la membrane plasmique des fibroblastes et libérer le contenu intracellulaire. Les 500 µl récupérés, contenant l'ascorbate-$^{14}$C intracellulaire, sont concentrés 10 fois et analysés par H.P.L.C.

*f. Dosage protéique*

Le dosage des protéines est réalisé par la méthode BCA (acide bicinchoninique, Sigma) sur le tapis de cellules perméabilisées de chaque puits, après solubilisation par de la soude 0,1 M. Les DO lues à 570 nm sont converties en ng de protéines à l'aide d'une gamme étalon de sérum albumine bovine (Sigma). Cette valeur permet d'exprimer la quantité d'ascorbate par µg de protéines.

*g. Dosage de l'ascorbate- $^{14}$C par H.P.L.C.*

L'ascorbate -$^{14}$C cellulaire est identifié par son temps de rétention identique à celui de la source radioactive -$^{14}$C initiale. La quantification s'effectue à l'aide d'un détecteur de radioactivité (Berthold) par intégration du pic correspondant.

La colonne utilisée est une colonne silice -NH$_2$ protégée par une colonne de garde remplie avec la même phase. L'éluant est composé d'acétonitrile gradient grade (Merck) 75 % (v/v) dans un tampon phosphate à pH = 5,95.

Les bilans de comptage de la radioactivité, contenu intracellulaire + extracellulaire sont d'environ 95%.

h. *Expression des résultats et interprétation statistique*

Les quantités d'ascorbate-$^{14}$C (noté Asc-$^{14}$C) intracellulaires sont exprimées en pmolc par μg de protéines après incubation de 2 h. Un calcul complémentaire permettant d'exprimer l'activité du produit (en %) a été effectué comme suit :

$$\left(\frac{\text{Asc}^{-14}\text{C des FNH traités - Asc }^{-14}\text{C des FNH témoins}}{\text{Asc-}^{14}\text{ C des FHN témoins}}\right) \times 100$$

Les résultats obtenus sur les cultures traitées et témoins sont comparés par le test t de Student non apparié. Les valeurs de p obtenues figurent dans les tableaux de résultats ci-dessous; p = 0.05 est fixée comme seuil de significativité.

5.2. <u>Résultats</u>

On a vérifié les propriétés de l'extrait méthanolique I$_3$ en le testant à trois concentrations et en travaillant avec 6 cultures témoins et 6 cultures traitées. Au préalable, on a vérifié l'absence d'effet propre du DMSO, excipient de solubilisation de l'extrait, sur les paramètres mesurés.

a. *Effet du DMSO sur le transport de l'ascorbate -$^{14}$C.*

L'effet du DMSO en ce qui concerne l'ascorbate figure dans le tableau 2 ci-dessous:

Tableau 2 :

| Contenu intracellulaire en ascorbate $^{-14}$C | | | |
|---|---|---|---|
| Traitement des cellules | Ascorbate intra.Cell pmole/μg prot/2 h | % d'activité | Significativité(*) |
| Témoin | 1,51 +/- 0,46 | | |
| + 0,1 % DMSO | 1,71 +/- 0,17 | (pas d'effet significatif) | NS( p=0,541) |

\* Comparaison effectuée entre 3 témoins et 3 traités. NS = non significatif (test de Student , seuil p < 0,05)

Conclusion : Le DMSO à 0,1 % v/v ne modifie pas de manière significative le contenu intracellulaire en ascorbate $^{14}$C.

b. *Effet de l'extrait I$_3$ sur le transport d'ascorbate $^{14}$C*

Le tableau 3 ci-dessous donne les résultats obtenus dans le cas du traitement par l'extrait I$_3$ en solution dans le DMSO.

Tableau 3 :

| Contenu intracellulaire en ascorbate$^{-14}$C | | | |
|---|---|---|---|
| Traitement des cellules | Ascorbate intra.Cell. pmole/μg prot/2h | % de stimulation | Significativité(∗) |
| + 0,1 % DMSO | 1,51 +/- 0,34 | | |
| I$_3$ 1 μg/ml | 2,35 +/- 0,45 | + 55 | S(p=0,005) |
| I$_3$ 2,5μg/ml | 2,30 +/- 0,36 | + 52 | S(p=0,003) |
| I$_3$ 5 μg/ml | 2,69 +/- 0,12 | + 78 | S(p=0,0001) |

S : significativité (Test de Student, seuil : p < 0,05)

∗ Comparaison effectuée entre 6 témoins et 6 traités

Conclusion:

Cette expérience montre clairement l'augmentation du contenu intracellulaire en ascorbate $^{14}$C des cellules traitées avec l'extrait $I_3$ (tableau 3). Cette augmentation est importante (+ 55 %) dès la concentration de 1 µg/ml et est statistiquement significative (p = 0,005). Aux concentrations de 2,5 et 5 µg/ml, l'extrait $I_3$ augmente également de manière significative (p = 0,003 et p = 0,0001 respectivement) le contenu intracellulaire en ascorbate-$^{14}$C (52 % et 78 % respectivement), le maximum d'effet se situant à la concentration de 5 µg/ml.

Ainsi les extraits de Bertholletia favorise très nettement l'incorporation de la vitamine C dans les fibroblastes.

## Exemple 6

| Composition de soin cosmétique contre le relâchement cutané | |
| --- | --- |
| Extrait méthanolique de péricarpe de noix de Bertholletia excelsa (exemple 2) | 0,2 g |
| Asiaticoside | 3 g |
| Palmitate de Vitaminc A | 0,01 g |
| Extrait de protéine de blé | 2 g |
| Excipient pénétrant et parfumé sous forme d'émulsion huile dans l'eau        qsp | 100 g |

Cette composition combat le relâchement de la peau et restaure sa fermeté. Appliquée sur les zones du buste, elle retend les tissus, leur donnant un plus bel aspect dès six semaines de traitement quotidien.

## Exemple 7

### Gel tenseur anti-rides pour le visage

| Extrait de Bertholletia excelsa (exemple 1) | 0,3 g |
| --- | --- |
| Palmitate de Vitamine A | 0,06 g |
| Acétate de Vitamine E | 0,1 g |
| Acide lactique | 1,5 g |
| Acide glycolique | 0,2 g |
| Ethanol | 5 g |
| Excipient gel        qsp | 100 g |

Ce gel peut être utilisé en applications locales sur les zones du cou et du front, avec massage jusqu'à pénétration complète. Dès six semaines, la peau est moins ridée et plus tonique. Son grain est plus fin, et le visage plus lumineux.

## Exemple 8

### Fond de teint traitant

| Extrait aqueux de péricarpe de noix de Bertholletia excelsa (exemple 3) | 0,3 g |
| --- | --- |
| Extrait de Centella asiatica | 0,2 g |
| Céramides de blé | 0,2 g |
| Protéines de blé | 3 g |
| Excipient coloré fluide        qsp | 100 g |

Ce maquillage de soin contribue à donner immédiatement un effet tenseur perceptible et agit également en profondeur sur les zones traitées ayant tendance au relâchement.

Exemple 9

Emulsion dermatologique

| Extrait de Bertholletia excelsa (exemple 1) | | 0,5 g |
|---|---|---|
| Palmitate d'ascorbyle | | 0,5 g |
| Excipient émulsionné sans parfum | qsp | 100 g |

Cette préparation dermatologique pourra être utilisée en applications locales sur les zones à traiter dans le cas d'épidermolyse bulleuse. Des signes nets d'amélioration cliniques apparaîtront. Cette composition devra cependant n'être utilisée que sur prescription et surveillance médicale stricte.

**Revendications**

1.  Utilisation d'un extrait de Bertholletia, en particulier d'un extrait de Bertholletia excelsa comme agent cosmétique destiné à stimuler la synthèse du collagène VII et/ou à favoriser l'incorporation de la vitamine C dans les cellules de la peau, en particulier dans les fibroblastes.

2.  Utilisation selon la revendication 1, caractérisée en ce que ledit extrait est utilisé pour stimuler la synthèse du collagène VII.

3.  Utilisation selon la revendication 1, caractérisée en ce que ledit agent cosmétique est utilisé pour favoriser l'incorporation de la vitamine C dans les cellules de la peau, en particulier dans les fibroblastes.

4.  Utilisation selon l'une des revendications précédentes, caractérisée en ce que ledit agent cosmétique est incorporé dans une composition à usage topique contenant en outre de la vitamine C ou un dérivé d'acide ascorbique.

5.  Utilisation selon l'une des revendications 1 à 4, caractérisée en ce que ledit agent cosmétique est incorporé dans une composition cosmétique à une concentration comprise entre 0,0001 % et 1 % en poids par rapport au poids total de ladite composition, de préférence entre 0,01 % et 0,25 %.

6.  Utilisation d'un extrait de Bertholletia, en particulier d'un extrait de Bertholletia excelsa, pour la préparation d'une composition pharmaceutique, notamment dermatologique destinée à traiter les pathologies liées à une déficience de la jonction dermo-épidermique, en particulier celles liées à une déficience de la synthèse du collagène VII.

7.  Utilisation selon la revendication 6, caractérisée en ce que ladite composition est utilisée pour le traitement de l'épidermolyse bulleuse.

8.  Utilisation d'un extrait de Bertholletia, en particulier d'un extrait de Bertholletia excelsa, pour la fabrication d'une composition pharmaceutique, notamment dermatologique destinée à traiter les pathologies liées à une déficience de l'incorporation de la vitamine C dans les cellules de la peau, en particulier dans les fibroblastes.

9.  Utilisation selon l'une des revendications 6 à 8, caractérisée en ce que la composition pharmaceutique contient en outre de la vitamine C ou un dérivé d'acide ascorbique.

10. Utilisation selon l'une des revendications 6 à 9, caractérisée en ce que ladite composition pharmaceutique contient une concentration d'extrait de Bertholletia comprise entre 0,0001 % et 1 % en poids par rapport au poids total de ladite composition, de préférence entre 0,01 % et 0,25 %.

11. Utilisation selon l'une des revendications 5 à 10, caractérisée en ce que ladite composition cosmétique ou pharmaceutique contient en outre une substance active choisie parmi le groupe constitué par l'acide madécassique, l'acide asiatique, le madécassoside, l'asiaticoside, l'alpha-1-protéinase inhibiteur, les inhibiteurs de collagénase, tels que l'acide rétinoïque, les inhibiteurs d'élastase, la lysine, la proline, le 2-oxoglutarate, le ginsénoside $R_0$, la vitamine D et ses dérivés, les ecdystéroïdes, en particulier la bêta-cedysone et la vitamine E.

**12.** Utilisation d'un extrait de Bertholletia, en particulier d'un extrait de Bertholletia excclsa, dans un milieu de culture de cellules, en particulier de cellules de la peau humaine, notamment de fibroblastes et de kératinocytes, comme agent destiné à stimuler la synthèse du collagène VII à partir desdites cellules et/ou comme agent destiné à favoriser l'incorporation de la vitamine C dans lesdites cellules.

**13.** Utilisation selon la revendication 12 pour la préparation d'une peau reconstituée;

**14.** Utilisation selon l'une des revendications 12 ou 13, caractérisée en ce que l'on introduit de 0,1 µg/ml à 10 µg/ml d'extrait de Bcrtholletia dans ledit milieu de culture.

**15.** Utilisation selon l'une des revendications 1 à 14, caractérisée en ce que l'extrait de Bertholletia précité est obtenu par extraction par un solvant polaire, avantageusement choisi dans le groupe constitué par l'eau, les alcools comportant de préférence de 1 à 4 atomes de carbone, tels que méthanol ou éthanol, les solvants chlorés comportant de préférence 1 ou 2 atomes de carbone tels que le chloroforme ou le dichlorométhane, les esters organiques comportant de préférence de 3 à 6 atomes de carbone et d'un solvant mixte à base d'un mélange quelconque des solvants précités.

**16.** Utilisation selon la revendication 15, caractérisée en ce que le solvant polaire est choisi dans le groupe constitué par l'eau, le méthanol, l'éthanol, et un solvant mixte à base d'un mélange quelconque des solvants précités.